# DEMANDE DE BREVET EUROPEEN

(11) **EP 4 371 414 A1**
(43) Date de publication de la demande: **22.05.2024**
(21) Numéro de dépôt: 23210955.3
(22) Date de dépôt: 20.11.2023
(51) Int. Cl.: A21D 2/26, A21D 2/36, A21D 10/00, A23L 5/10, A23L 5/30, A23L 33/18, A23L 33/185, A23L 33/19, A23L 33/21, A23L 33/00, A61P 1/00

(54) **PRODUITS NUTRITIFS ADAPTÉS AUX PERSONNES SOUFFRANT DE DYSPHAGIE**

(30) Priorité: 21.11.2022 FR 2212090
(71) Demandeur: Senes Solutions, 69530 Brignais (FR)
(72) Inventeur: THUBÉ, Frédéric, 69110 Sainte Foy-Lès-Lyon (FR); JOLIVET LAPIERRE, Elise, 44240 Sucé-sur-Erdre (FR); GARNIER, Julien, 69340 FRANCHEVILLE (FR); BENNANI, Sarah, 69230 Saint-Genis-Laval (FR)
(74) Mandataire: Aquinov

(57) **Abrégé**

La présente invention concerne l'amélioration et le maintien de la santé de personnes souffrant de troubles de la déglutition, de la mastication, en particulier de dysphagie, en fournissant une nutrition particulièrement adaptée. En particulier, l'invention concerne un produit alimentaire reconstitué dont la texture est améliorée, ainsi que son procédé de préparation, et une poudre alimentaire destinée à la reconstitution du produit selon l'invention.

## Description

### Domaine technique

L'invention concerne l'amélioration et le maintien de la santé de personnes souffrant de troubles de la déglutition et/ou de la mastication, notamment de dysphagie, en fournissant une nutrition particulièrement adaptée. En particulier, l'invention concerne un produit alimentaire reconstitué dont la texture est plus adaptée aux personnes souffrant de troubles de la déglutition et/ou mastication, ainsi que son procédé de préparation, et une poudre alimentaire destinée à la reconstitution du produit selon l'invention.

### Etat de l'art

La déglutition désigne le mécanisme d'absorption, notamment du bol alimentaire, de la bouche, puis du pharynx et de l'œsophage jusqu'à l'estomac. Ce mécanisme complexe met en jeu plusieurs étapes avec la participation de différents organes ainsi que de nombreux muscles et nerfs. Une phase orale d'introduction des aliments en bouche, consiste à mastiquer et insaliver les aliments permettant la cohésion du bol alimentaire.

Or, les personnes âgées, notamment, sont souvent sujettes à des problèmes bucco-dentaires entraînant des difficultés plus ou moins importantes à mastiquer correctement les aliments. Le processus de vieillissement entraîne également une sécheresse buccale et donc, une baisse importante de la capacité à insaliver les aliments, pouvant entraîner un risque important de fausse route.

Ainsi, les personnes présentant des troubles de la déglutition montrent des difficultés à transporter les aliments de la bouche à l'estomac, sans risques de fausse route. Les personnes souffrant de dysphagie présentent, en sus, une sensation de gêne ou d'inconfort au cours de la déglutition avec des difficultés à initier la déglutition. En Europe, environ 30% des personnes de plus de 75 ans sont atteintes de dysphagie avec plus de 50% des personnes âgées institutionnalisées.

Les personnes atteintes de ces troubles présentent une diminution des apports alimentaires, une appréhension lors de l'alimentation, voire le refus des prises alimentaires. Très souvent, ces troubles entraînent une perte de poids importante, un risque de dénutrition accru, des infections pulmonaires, voire conduisent au décès de la personne.

Ainsi, la prise en charge de ces personnes nécessite d'adapter l'offre alimentaire, en particulier en proposant des aliments adaptés, par exemple via une modification de leurs textures. Toutefois, le changement des habitudes alimentaires, par exemple par l'apport d'aliment à la texture modifiée, peut entraîner des conséquences psychologiques sur les personnes car le plaisir de manger est intimement lié au visuel et à la texture des aliments. Le phénomène de rejet des repas peut alors être aggravé par la baisse du plaisir de manger.

Le pain est un produit largement utilisé pour accompagner les repas, notamment en France. Il est consommé pour sa texture alvéolée et son goût particulier. D'un point de vue nutritionnel, le pain est également l'un des seuls produits dont l'être humain pourrait se contenter pour subsister, dû à sa composition adaptée aux besoins journaliers, 100 g de pain apporte environ 55 g de glucides, 0,70 g de lipides, 7,5 g de protides, des sels minéraux et des vitamines.

Malheureusement, lorsque les individus souffrent de troubles de la mastication, déglutition, en particulier de dysphagie, par exemple liés au vieillissement, à la prise de médicaments, ou à des pathologies telles que des maladies oto-rhino-laryngologiques ou oesophagiennes, ou d'une maladie dégénérative, par exemple Parkinson, le pain est très souvent abandonné, bouleversant ainsi, les habitudes alimentaires.

Les solutions apportées pour remplacer le pain peuvent reposer sur l'utilisation de pain de mie trempé dans du lait ou autre liquide pour l'humidifier et le ramollir. Toutefois, cette solution n'est pas adaptée, notamment car elle vise des personnes ayant encore la capacité de consommer des aliments avec néanmoins des difficultés pour mastiquer et/ou les déglutir. De plus, visuellement et gustativement, cette solution ne peut remplacer le pain.

D'autres produits tentent de proposer une texture molle et proche du pain de mie ou de la brioche et facile à mastiquer, par exemple le pain G-Nutrition^{®} qui est un pain brioché dédié aux personnes dénutries ayant des difficultés mineures à mâcher certains aliments. Or, là encore, ce produit n'est pas adapté aux personnes atteintes de dysphagie.

Il existe donc un besoin pour un nouveau produit nutritionnel adapté pour les personnes souffrant de troubles de la déglutition, plus particulièrement des personnes atteintes de dysphagie. C'est l'objectif de la présente invention.

### Résumé de l'invention

Pour répondre à ce besoin, l'invention propose un produit nutritionnel reconstitué à partir d'une poudre mélangée dans un liquide. Pour y répondre, ladite poudre comprend un mélange particulier de fibres permettant, après reconstitution dans ledit liquide, d'obtenir un produit alimentaire reconstitué particulièrement adapté pour les personnes souffrant de troubles de la déglutition et/ou de la mastication, en particulier de dysphagie.

Le produit nutritionnel selon l'invention vise, avantageusement, un ou plusieurs produits de boulangerie (pain, brioche, pain de mie, bretzel, bagel ...), viennoiserie (croissant, pain au chocolat, ...), mais également de pâtisserie (muffins, cake, pancake, financier, madeleine, scones, churros, beignets, donuts...). La texture de ces produits peut être modifiée tout en conservant les propriétés nutritionnelles et organoleptiques afin d'être consommés par des personnes qui présentent des risques de fausses routes ou atteintes de troubles de la déglutition. Aussi, le produit nutritionnel selon l'invention est préférentiellement un produit de panification, tel qu'un produit de boulangerie, de viennoiserie ou de pâtisserie.

Avantageusement, la texture dite modifiée est obtenue en adaptant la composition d'une part, et le procédé de cuisson d'autre part. De façon particulièrement avantageuse, une texture dite normale peut également être obtenue par l'utilisation d'un procédé de cuisson usuel en boulangerie.

Ainsi, le produit nutritionnel selon l'invention est particulièrement adapté aux personnes présentant des risques de fausses routes, ou atteintes de troubles de la déglutition et/ou de la mastication, et/ou de dysphagie, mais également aux personnes à risque de dénutrition ou dénutries. En effet, une portion de produit nutritionnel selon l'invention peut apporter entre 8 et 20 % des apports protéiques journaliers.

Ainsi, l'invention concerne selon un premier aspect un produit nutritionnel pour son utilisation dans la prévention et/ou le traitement des troubles de la mastication et/ou de la déglutition et/ou de la dénutrition, comprenant :
- (i) un liquide choisi parmi l'eau, le lait et une boisson végétale, et
- (ii) un mélange comprenant :
   ▪ au moins une fibre fonctionnelle,
   ▪ au moins une protéine foisonnante,
   ▪ au moins une farine de céréales, et
   ▪ du blanc d'oeuf.

Selon un second aspect, la présente invention se rapporte également à une poudre alimentaire destinée à être reconstituée dans un liquide, c'est à dire un mélange, comprenant :
- au moins une fibre fonctionnelle,
- au moins une protéine foisonnante,
- au moins une farine de céréales, et
- du blanc d'oeuf.

Avantageusement, la protéine foisonnante est choisie parmi un peptide hydrolysé de protéine végétale, une protéine d'origine animale ou végétale, et leurs combinaisons. Plus préférentiellement, le peptide hydrolysé est un peptide hydrolysé de protéine de pois et la protéine d'origine animale est une protéine de lait. De façon particulièrement avantageuse, la poudre comprend au moins deux protéines foisonnantes, en l'espèce un peptide hydrolysé de protéine de pois et une protéine de lait.

Préférentiellement, le rapport peptides hydrolysés de protéine de pois et protéine de lait est compris entre 1 et 2, permettant d'obtenir une texture mousseuse, particulièrement adaptée dans le contexte de l'invention.

Le produit selon l'invention comprend au moins une fibre fonctionnelle, préférentiellement celle-ci présente une capacité de rétention d'eau comprise entre 500 et 3500%, plus préférentiellement, la fibre fonctionnelle est choisie parmi la fibre de blé, de pois, d'avoine, d'agrume, de lin, de carotte, psyllium et leurs combinaisons. La fibre fonctionnelle permet de retenir les molécules d'eau, permettant ainsi au produit nutritionnel selon l'invention d'augmenter son volume et de limiter la présence d'un effet collant en bouche, préjudiciable aux personnes souffrant de troubles de la mastication et/ou de la déglutition.

Enfin, le produit nutritionnel selon l'invention, ainsi que la poudre selon l'invention comprennent également du blanc d'oeuf et au moins une farine de céréales, préférentiellement une farine de céréales présentant une capacité de rétention d'eau d'au moins 100%, avantageusement, la farine de céréales est une farine de maïs, de façon particulièrement avantageuse une farine de maïs fonctionnalisée.

Le produit nutritionnel selon l'invention, ainsi que la poudre selon l'invention peuvent également comprendre d'autres ingrédients, préférentiellement au moins un ingrédient supplémentaire choisi parmi une gomme alimentaire, une fibre nutritionnelle, un levain, un arôme, de l'amidon, du malt d'orge, de la poudre à lever, un acide aminé, une vitamine, un minéral, un extrait végétal, des omégas 3 et leurs combinaisons. Etant entendu, que le produit nutritionnel selon l'invention peut également comprendre d'autres ingrédients à vocation nutritionnelle ou substance nutritionnelle.

Avantageusement, la poudre est particulièrement adaptée dans le contexte de l'invention en ce qu'elle permet de faciliter sa conservation sur plusieurs mois (de l'ordre de 4 à 12 mois), mais également d'assurer et conserver les qualités organoleptiques de la poudre tout au long du temps de stockage, et par conséquent du produit alimentaire reconstitué. La poudre selon l'invention, étant donnée sa composition particulière est particulièrement adaptée pour pouvoir être reconstituée dans un liquide, permettant l'obtention du produit nutritionnel selon l'invention.

Le produit sous forme de poudre est ainsi, reconstitué dans un liquide. Lors de la reconstitution du produit, des forces de cisaillement importantes sont appliquées au mélange (poudre selon l'invention et solution liquide) pour activer la fonctionnalité des fibres fonctionnelles présentes dans le mélange, et ainsi obtenir un produit alimentaire reconstitué adapté pour les personnes souffrant de troubles de la mastication et/ou de la déglutition.

Selon un objet préféré de l'invention, le produit alimentaire reconstitué est préparé/obtenu par un organe de mélange configuré pour générer une force de cisaillement comprise entre 3000 et 12500 rpm, adaptée pour mélanger le liquide (i) et le mélange (ii), en l'espèce la poudre, comprenant au moins la fibre fonctionnelle, la protéine foisonnante, la farine de céréales et le blanc d'oeuf.

Le mélange ainsi obtenu peut alors être introduit, dans un four vapeur, un micro-ondes ou au bain-marie pour une cuisson en chaleur humide afin d'obtenir un produit en texture modifiée ou introduit dans un four en chaleur sèche pour obtenir une texture normale.

Préférentiellement, le produit alimentaire reconstitué selon l'invention est obtenu par cuisson vapeur et présente une texture modifiée de grade inférieur ou égal à 6, préférentiellement de grade 5 ou 6, plus préférentiellement de grade 5, mesuré selon l'échelle IDDSI (International Dysphagia Diet Standardisation Initiative). Selon une variante, le produit alimentaire reconstitué selon l'invention est obtenu par cuisson au bain marie ou par cuisson micro-ondes, avantageusement au bain marie à 90°pendant 20-25mn pour une portion de l'ordre de 32g.

Le produit alimentaire est alors, particulièrement adapté aux personnes souffrant de troubles de la déglutition et/ou de la mastication, ou souffrant de dénutrition, plus préférentiellement de dysphagie. Ainsi, l'invention se rapporte également à son utilisation dans la prévention et/ou le traitement de la dysphagie et/ou de la dénutrition et/ou des troubles de la déglutition et/ou de la mastication.

Le procédé de panification est composé classiquement de trois grandes étapes : le pétrissage, la fermentation/la poussée et la cuisson. Or, de façon avantageuse, la présente invention se rapporte à un procédé modifié et rapide sans fermentation et/ou poussée.

Ainsi, selon un autre aspect, l'invention concerne un procédé de préparation du produit nutritionnel reconstitué selon l'invention comprenant les étapes suivantes :
- mélanger (i) la poudre comprenant au moins une fibre fonctionnelle, au moins une protéine foisonnante, au moins une farine de céréales ayant, préférentiellement une capacité de rétention d'eau d'au moins 100%, du blanc d'oeuf, et (ii) un liquide choisi parmi l'eau, le lait et une boisson végétale, par un organe de mélange configuré pour générer une force de cisaillement comprise entre 3000 et 12500 rpm, pendant une durée comprise entre 30 secondes et 2 minutes,
- verser dans un moule le mélange obtenu à l'étape a), et
- cuire au moyen d'un four vapeur ou d'un four à chaleur sèche.

La cuisson est avantageusement mise en oeuvre au moyen d'un four vapeur, d'un bain marie, d'un micro-ondes ; ou d'un four à chaleur sèche, c'est-à-dire respectivement en phase humide ou en phase sèche. La phase humide permet d'une part d'être particulièrement adaptée aux personnes sujettes aux fausses routes, souffrant de troubles de la déglutition et/ou de la mastication, mais également de la dysphagie, et d'autre part, de faciliter la préparation, sur place dans les établissements médico-sociaux et sanitaires avec du matériel classique de restauration collective.

D'autres caractéristiques et avantages ressortiront de la description détaillée de l'invention, des exemples et des figures qui vont suivre.

### Brève description des Figures

[Figure 1] est un diagramme des textures représentant les différents grades définis par l'IDDSI, version 2022, afin de décrire et définir les aliments de texture modifiée et les liquides épaissis utilisés auprès de personnes dysphagiques de tous âges. Il est composé d'un continuum de 8 niveaux (du grade 0 à grade 7).
[Figure 2a] est une image représentant une section transversale d'une portion de pain en texture modifié selon l'exemple 2, comprenant des grandes et petites alvéoles caractéristiques d'un pain classique.
[Figure 2b] est une image traitée par le logiciel ImageJ de l'image représentée en Figure 2a.
[Figure 3a] est une image représentant une section transversale d'une portion de pain en texture normale selon l'exemple 3, comprenant une majorité de petites alvéoles.
[Figure 3b] est une image traitée par le logiciel ImageJ de l'image représentée en Figure 3a.
[Figure 4a] est une image représentant une section transversale de pain du produit G-Nutrition^{®}.
[Figure 4b] est une image traitée par le logiciel ImageJ de l'image représentée en Figure 4a.
[Figure 5a] représente les résultats du test IDDSI à la fourchette selon les critères définis pour le grade 5.
[Figure 5b] représente une image du test IDDSI à la cuillère inclinée selon les critères définis dans le grade 5.

### Description détaillée de l'invention

### Définitions

Par « amidon » au sens de l'invention, on entend un glucide complexe composé d'unités de D-glucose et composé de deux polymères : l'amylose et l'amylopectine. L'amidon au sens de l'invention est un amidon modifié par voie physique ou chimique, préférentiellement un amidon de blé modifié par voie physique. Cette modification, avantageusement physique a pour objectif de rendre l'amidon natif plus soluble en mettant en oeuvre un traitement à la chaleur en milieu humide. Ce traitement va notamment permettre un gonflement différé de cet ingrédient afin de faciliter l'hydratation des autres ingrédients de la formule tels que les fibres ou les protéines.

Par « boisson végétale » ou « lait végétal » au sens de l'invention, on entend des boissons exclusivement d'origine végétale, notamment obtenues à partir de céréales ou pseudo céréales (avoine, riz, épeautre, quinoa, orge...), d'oléagineux (noisette, amande, noix) ou de légumineuses (soja, pois chiches), auxquels on ajoute de l'eau, ayant le même aspect et la même couleur que le lait d'origine animale. Dans le contexte de l'invention, la boisson végétale est un substitut au lait. Avantageusement, la boisson végétale ne comprend pas ni lactose, ni cholestérol et est moins riche en gras. Préférentiellement la boisson végétale peut être choisie parmi le lait de riz, le lait d'avoine, le lait d'amande, le lait de soja, le lait de soja, le lait de noisette, le lait de chanvre, le lait d'épeautre, le lait de châtaigne, et le lait de coco.

Par « fibre fonctionnelle » au sens de l'invention, on entend des fibres alimentaires qui sont des polymères glucidiques composées de plus de trois unités monomériques, non digérées ni absorbées dans l'intestin grêle humain et avec une fermentation complète ou partielle dans le gros intestin procurant des effets physiologiques bénéfiques. Elles peuvent être solubles ou insolubles et avantageusement choisies parmi les fibres de soja, haricots, maïs, lentilles, pois, riz complet, pruneaux, flocons de son, blé, pâtes complètes, avoine, agrume, lin, psyllium, abricots, artichaut, carotte, framboises et les gommes alimentaires telles que la gomme de xanthane. Les fibres fonctionnelles vont apporter une texture au produit et permettent de stabiliser les alvéoles dû à leurs propriétés rhéologiques. Les fibres ont ainsi la capacité de se disperser, de s'hydrater puis d'augmenter la viscosité du mélange pour limiter le drainage du liquide évitant que la coalescence des bulles d'air. Aussi, les fibres fonctionnelles ont une capacité de rétention d'eau importante.

Par « capacité de rétention d'eau » au sens de l'invention, on entend l'aptitude d'une quantité de fibres ou de farine à retenir une quantité d'eau lorsqu'elle est soumise à des conditions de température, de temps d'agitation, de vitesse ou de temps de centrifugation. Cette propriété va permettre aux fibres en solution de s'hydrater et de gonfler. Lors du gonflement, le volume du mélange va augmenter entraînant une augmentation de la viscosité jusqu'à la création d'un réseau épaissi ou gélifié.

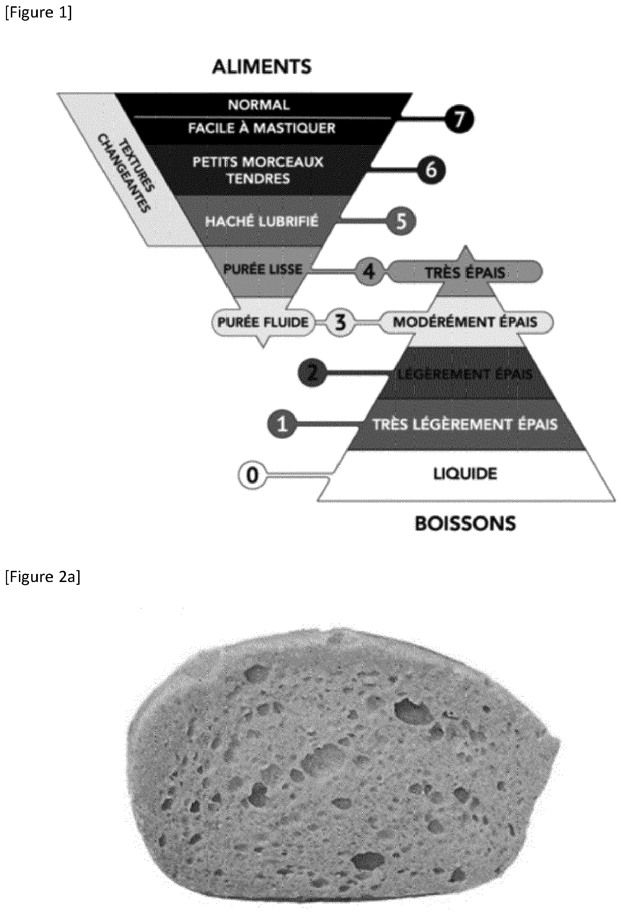

Par « grade mesuré selon l'échelle IDDSI » au sens de l'invention, on entend, une méthode de mesure internationale standardisée des textures adaptées à la dysphagie, initiée en 2013, afin de développer une terminologie, universelle et standardisée, permettant de décrire et définir les aliments de texture modifiée et les liquides épaissis utilisés auprès de personnes dysphagiques de tous âges. L'IDDSI (International Dysphagia Diet Standardisation Initiative) a ainsi abouti à un diagramme final des textures en dysphagie, composé d'un continuum de 8 niveaux (0 à 7) [Figure 1]. Ces niveaux sont identifiés par des numéros, des libellés et un code couleur (Cichero, J. A. Y., et al. (2016). Development of International Terminology and Définitions for Texture-Modified Foods and Thickened Fluids Used in Dysphagia Management: The IDDSI Framework. Dysphagia, 32(2), 293 314 (doi.org/10.1007/s00455-016-9758-y)).

Par « lait » au sens de l'invention, on entend une boisson exclusivement d'origine animale, en particulier il s'agit d'un liquide biologique comestible généralement de couleur blanchâtre produit par les glandes mammaires des mammifères femelles. Le lait peut être choisi parmi le lait de vache, le lait de chèvre, le lait de buffle, le lait de chamelle, et le lait d'ânesse, plus préférentiellement le lait de vache.

Par « peptide hydrolysé » au sens de l'invention, on entend des peptides ayant subi une hydrolyse, par exemple une hydrolyse chimique et/ou en enzymatique. Les peptides sont préférentiellement des peptides d'origine végétale ou animale ayant subi une hydrolyse c'est-à-dire des protéines végétales ou animales hydrolysées. Des peptides particulièrement d'intérêt dans le contexte de l'invention sont des peptides de pois hydrolysés, particulièrement riche en lysine.

Par « produit nutritionnel » ou « produit alimentaire » au sens de l'invention, on entend une substance qui peut être utilisée ou préparée pour fournir une nourriture à un être humain ou un animal. En particulier, dans le contexte de l'invention, le produit nutritionnel est reconstitué à partir d'une poudre comprenant plusieurs fibres et protéines adaptés à la nutrition, notamment humaine, dans un liquide permettant d'obtenir une texture particulièrement adaptée pour les personnes souffrant de dénutrition, de troubles de la mastication et/ou de la déglutition, en particulier les personnes dysphagiques. Le produit nutritionnel selon l'invention est un produit de panification, tel qu'un produit de boulangerie, de viennoiserie ou de pâtisserie. Ledit produit permettant également de couvrir les besoins nutritionnels journaliers, préférentiellement la quantité de fibres et de protéines est de 2 à 4 fois supérieure par rapport à une portion équivalente d'un pain traditionnel. A titre d'exemple, le produit selon l'invention apporte 10 à 15% des besoins nutritionnels en protéines des personnes âgées non dénutries, soit un besoin de 1g/kg de poids corporel/jour. De façon avantageuse, le produit nutritionnel au sens de l'invention n'est pas une boisson.

Par « protéine de lait » ou « protéines laitières » au sens de l'invention, on entend un type de protéine dérivé du lait obtenu à partir des protéines de lait totales (caséines + sériques) ou uniquement sériques, suivi d'une étape de purification/concentration/séchage. Dans le contexte de l'invention, les protéines de lait sont avantageusement obtenues après une étape d'ultrafiltration, afin d'éliminer l'eau, une étape de chauffage du rétentat à une température d'au moins 100°C et enfin une étape de refroidissement.

Par « protéine foisonnante » au sens de l'invention, on entend des protéines présentant des propriétés foisonnantes, c'est-à-dire ayant la capacité à former une mousse ou une émulsion avec l'air, préférentiellement elles agissent comme agent de foisonnement pour former un film protéique à l'interface des bulles d'air afin de les stabiliser et de faciliter l'incorporation d'air dans la phase continue. Dans le contexte de l'invention, les protéines foisonnantes peuvent également être des protéines du blanc d'oeuf ou des protéines laitières sériques. Préférentiellement, les protéines foisonnantes sont des peptides hydrolysés de protéine végétale et/ou des protéines d'origine animale, plus préférentiellement des peptides de pois et/ou des protéines de lait et/ou du blanc d'oeuf.

Par « prévention » ou « prophylaxie » au sens de l'invention, on entend la réduction à un degré moindre du risque ou de la probabilité d'occurrence d'un phénomène donné, c'est-à-dire, dans le contexte de la présente invention, de la survenance d'un trouble de la mastication et/ou de la déglutition et/ou de la dénutrition, préférentiellement de la dysphagie.

Par « traitement » au sens de l'invention, on entend une diminution de la progression, une stabilisation, une inversion ou régression, voire une interruption ou inhibition d'un trouble ou d'un symptôme, préférentiellement d'un trouble de la mastication et/ou de la déglutition et/ou de la nutrition, plus préférentiellement de la dysphagie.

### Produit nutritionnel selon l'invention

La présente invention a donc pour objet un produit nutritionnel comprenant :
- (i) un liquide choisi parmi l'eau, le lait et une boisson végétale
- (ii) un mélange comprenant :
   ▪ au moins une fibre fonctionnelle,
   ▪ au moins une protéine foisonnante,
   ▪ au moins une farine de céréales, préférentiellement une farine de céréales ayant une capacité de rétention d'eau d'au moins 100%, et
   ▪ du blanc d'oeuf.

De façon avantageuse, le produit nutritionnel est reconstitué à partir d'une poudre comprenant :
- au moins une fibre fonctionnelle,
- au moins une protéine foisonnante,
- au moins une farine de céréales, préférentiellement une farine de céréales ayant une capacité de rétention d'eau d'au moins 100%, et
- du blanc d'oeuf.

Ainsi, ledit produit nutritionnel selon la présente invention comprend avantageusement (i) le liquide et ladite poudre selon l'invention. Celui-ci est avantageusement préparé à partir de la poudre selon l'invention ou du mélange (ii) dans (i) le liquide.

La présente invention porte d'une part, sur la composition particulière du produit mettant en jeu l'association entre, notamment, des protéines ayant des propriétés foisonnantes et des fibres fonctionnelles ; et d'autre part, sur le procédé de préparation.

Ainsi, la poudre et le produit nutritionnel susceptible d'être obtenu à partir de la poudre comprend au moins une protéine foisonnante. Cette protéine présente des propriétés foisonnantes, c'est-à-dire ayant la capacité de stabiliser les bulles d'air en formant un film autour d'elles pour favoriser l'incorporation d'air et former une mousse ou une émulsion, permettant ainsi d'obtenir une texture mousseuse. Une telle texture est particulièrement d'intérêt, pour les personnes souffrant de problèmes de mastication et/ou déglutition, et par conséquent, les personnes souffrant de dysphagie.

Préférentiellement, la protéine foisonnante présente dans la poudre, mais également dans le produit nutritionnel selon l'invention, est choisie parmi un peptide hydrolysé de protéine végétale, une protéine d'origine animale ou végétale, et leurs combinaisons. Plus préférentiellement, un peptide hydrolysé de protéine végétale, et/ou une protéine d'origine animale. Selon une variante de l'invention, les protéines foisonnantes peuvent également être des protéines du blanc d'oeuf ou des protéines laitières sériques.

Selon un objet particulièrement préféré, la poudre, ainsi que le produit nutritionnel selon l'invention comprennent au moins deux protéines foisonnantes, avantageusement un peptide hydrolysé de protéine végétale et une protéine d'origine animale. Plus préférentiellement, le peptide hydrolysé est un peptide hydrolysé de protéine de pois et la protéine d'origine animale est une protéine de lait. En effet, une association entre les peptides hydrolysés de protéine de pois et les protéines laitières permet d'atteindre un taux de foisonnement important, avantageusement un taux compris entre 50 et 500%, particulièrement utile dans le contexte de l'invention.

Selon un autre objet préféré de l'invention, la sélection d'un rapport protéines de pois / protéines laitières particulier, permet à la fois d'assurer la texture la plus mousseuse possible du produit fini, tout en évitant d'avoir un arrière-goût amer dû à la présence des peptides hydrolysées de protéines de pois. Ainsi, la texture finale du produit nutritionnel selon l'invention est particulièrement adaptée lorsque ledit produit et la poudre présente un rapport peptides hydrolysés de protéine de pois et protéine de lait compris entre 1 et 2.

Plus préférentiellement, les peptides hydrolysés de protéine de pois représentent au plus 7% en poids du poids total de matière sèche et les protéines de lait représentent au plus 14% en poids du poids total de matière sèche.

La poudre et/ou le produit nutritionnel selon l'invention comprend également au moins une fibre fonctionnelle. De façon avantageuse, lors du mélange, sous l'effet du cisaillement apporté par l'organe de mélange, par exemple un robot-mixeur, la ou les fibres fonctionnelles vont avoir la capacité de retenir les molécules d'eau, permettant ainsi au produit nutritionnel selon l'invention d'augmenter de volume et d'empêcher la formation d'un effet collant en bouche en texture modifiée. Un effet collant en bouche, dans le contexte de l'invention, est particulièrement indésirable, ayant pour conséquence de coller les aliments en bouche, laisser des résidus susceptibles d'être dangereux pour des personnes souffrant déjà de tels troubles. Aussi, une telle texture n'est pas adaptée à ces personnes et notamment les personnes dysphagiques. A l'inverse, le produit selon l'invention se délite en bouche sous l'action de la langue, en particulier le produit s'écrase sous l'action de la langue.

Les fibres fonctionnelles contribuent à la stabilité de la mousse afin d'éviter la coalescence des bulles d'air. Enfin, elles permettent d'apporter de la fraicheur ainsi que du moelleux du produit fini, sans présenter ledit effet collant en bouche.

Les propriétés des fibres fonctionnelles sont d'autant plus efficaces lorsque le mélange est effectué sous l'effet d'un cisaillement mis en oeuvre après reconstitution du mélange en poudre dans le liquide. Avantageusement, les fibres fonctionnelles permettent d'augmenter la viscosité du mélange et de favoriser la rétention des bulles d'air. En outre, Le produit sera également légèrement visqueux en bouche avec l'ajout des fibres fonctionnelles facilitant le passage des morceaux consommés. Cet effet est encore amélioré lorsque le produit et/ou la poudre selon l'invention comprend au moins une gomme xanthane.

Selon un objet préféré, la quantité de fibre fonctionnelle est comprise entre 2 et 12% en poids du poids total de matière sèche, plus préférentiellement entre 5 et 10%.

Dans le contexte de l'invention, plusieurs fibres peuvent être utilisées telles que la fibre de blé, la fibre de pois, la fibre d'avoine, la fibre d'agrume, la fibre de lin, la fibre de soja, la fibre de carotte, ou encore la fibre de psyllium, y compris des gommes alimentaires telles que la gomme xanthane. Ainsi, la fibre fonctionnelle est préférentiellement choisie parmi la fibre de blé, de pois, d'avoine, d'agrume, lin, psyllium, soja, carotte et leurs combinaisons.

Des associations de plusieurs fibres sont également possibles, comme la fibre de blé et la fibre de psyllium. A titre d'exemple, la fibre de blé ou la fibre de pois peuvent être utilisée à hauteur de 2 à 12% en poids de matière sèche du produit.

De façon avantageuse, l'ajout de fibre d'acacia dans la poudre et/ou le produit nutritionnel permet d'apporter une source de fibres nutritionnelles solubles intéressante pour les personnes âgées souffrant de troubles du transit intestinal sans modifier les propriétés organoleptiques du produit nutritionnel.

La poudre et/ou le produit nutritionnel selon l'invention comprend également du blanc d'oeuf, celui-ci permet d'apporter des protéines de hautes qualités nutritionnelles et contribue également à la texture mousseuse du produit nutritionnel reconstitué. Préférentiellement, la quantité de blanc d'oeuf est comprise entre 3 et 25% en poids du poids total de matière sèche, plus préférentiellement entre 8 et 18%, en poids du poids total de matière sèche, permettant au produit nutritionnel d'être notamment tartinable ains que de contribuer à atteindre la texture alvéolée et mousseuse.

La poudre et/ou le produit nutritionnel comprend également de la farine de céréales ayant, préférentiellement une capacité de rétention d'eau d'au moins 100%. Préférentiellement, ladite farine de céréales est une farine de maïs, dites fonctionnelle. La farine de maïs fonctionnelle est une farine de maïs ayant subi un traitement physique permettant d'agir comme un épaississant naturel.

Préférentiellement, la quantité de farine de maïs est comprise entre 10 et 30% en poids du poids total de matière sèche, plus préférentiellement entre 15 et 30% en poids du poids total de matière sèche.

Selon un autre objet de l'invention, la poudre et/ou le produit nutritionnel comprend préférentiellement, au moins ingrédient supplémentaire choisi parmi une gomme alimentaire, une fibre nutritionnelle, un levain, un arôme, de l'amidon, du malt d'orge, de la poudre à lever, un acide aminé, une vitamine, un minéral, un extrait végétal et leurs combinaisons.

Le levain et les arômes apportent notamment le goût typique du pain, améliorant gustativement le produit nutritionnel selon l'invention. Par conséquent, les personnes redécouvrent le plaisir de manger qui est intimement lié au visuel et à la texture des aliments. Le phénomène de rejet des repas est alors fortement diminué.

En particulier, le goût de la croûte de pain doit pouvoir être perçu pour le produit en texture modifiée, et ce alors qu'un procédé de cuisson en phase humide ne permet habituellement pas de développer ces arômes caractéristiques, développés lors d'un procédé de cuisson en phase sèche. Afin d'apporter ces notes grillées et toastées, un arôme « croûte de pain » est préférentiellement ajouté entre 0,1% et 1% en poids du poids total de matière sèche.

L'amidon peut également être apporté jusqu'à, préférentiellement au plus 3% en poids du poids total de matière sèche, ce qui permet d'améliorer encore la texture en stabilisant les bulles d'air lors de la cuisson. En effet, l'amidon va gélatiniser et contribuer ainsi à rigidifier la membrane de protéines autour des bulles d'air pour obtenir une mousse dite fixe après cuisson, évitant d'avoir un produit nutritionnel en texture modifiée qui soit collant en bouche, c'est-à-dire présentant un effet collant en bouche, limitant le risque de fausses routes pour les personnes en ayant besoin.

Afin d'apporter le brunissement au produit en texture modifiée et également le goût acide du pain au levain, le produit comprend avantageusement un levain de blé. Celui-ci est encore plus avantageusement compris entre 2 et 8% en poids du poids total de matière sèche. Eventuellement, le produit comprend également du malt d'orge entre 1% et 3,5% en poids du poids total de matière sèche.

Préférentiellement le produit nutritionnel et/ou la poudre selon l'invention comprend de la poudre à lever, afin de faciliter sa mise en oeuvre. De façon particulièrement avantageuse, celle-ci est comprise entre 1 et 6% en poids du poids total de matière sèche.

D'autres ingrédients peuvent également être présents dans le produit nutritionnel tel qu'au moins un acide aminé, une vitamine, un minéral, un extrait végétal permettant ainsi de couvrir les besoins quotidiens des patients. Etant entendu, que le produit nutritionnel selon l'invention peut également comprendre d'autres ingrédients à vocation nutritionnelle ou substance nutritionnelle, tel que par exemple des omégas.

Ainsi, l'ensemble de ces ingrédients présents dans le produit nutritionnel selon l'invention et, selon un autre objet la poudre selon l'invention, en particulier, les fibres fonctionnelles, les protéines ayant des propriétés foisonnantes, le blanc d'oeuf et la farine de céréales fonctionnalisée ayant une capacité de rétention d'eau, permettent ensemble, de façon synergique, de stabiliser l'ensemble des particules en suspension et de stabiliser les bulles formées au cours de la reconstitution. Ces bulles vont donner une structure bien alvéolée similaire à un pain traditionnel. Aussi, le produit nutritionnel, une fois reconstitué présente les qualités nutritionnelles nécessaires ainsi qu'une texture, étant à la fois, proche des produits dits de boulangerie, et suffisamment modifiée pour obtenir une texture modifiée de grade 6 ou moins préférentiellement de grade 5 ou 6, plus préférentiellement de grade 5, mesuré selon l'échelle IDDSI.

Afin d'obtenir une telle texture modifiée, le produit nutritionnel reconstitué est préférentiellement préparé ou obtenu par un organe de mélange configuré pour générer une force de cisaillement comprise entre 3000 et 12500 rpm, adaptée pour mélanger (i) le liquide et les différents ingrédients nécessaires, soit (ii) le mélange ou la poudre comprenant au moins la fibre fonctionnelle, la protéine foisonnante, la farine de céréales et le blanc d'oeuf.

De façon encore plus préférentielle, le produit nutritionnel est obtenu par cuisson vapeur et présente ainsi, une texture modifiée de grade 6 ou moins, mesuré selon l'échelle IDDSI, préférentiellement de grade 5.

Le produit nutritionnel peut être indifféremment préparé par l'Homme du métier afin d'obtenir la texture d'intérêt, soit une texture choisie parmi une texture de grade 4, 5, et 6, mesuré selon l'échelle IDDSI, préférentiellement la texture modifiée est de grade 5.

L'échelle IDDSI est une méthode de mesure internationale standardisée des textures adaptées à la dysphagie. Celle-ci a été initiée en 2013, afin de développer une terminologie, universelle et standardisée, ainsi que des définitions décrivant les aliments de texture modifiée et les liquides épaissis utilisés auprès de personnes dysphagiques de tous âges, dans tout type de structures de soins, et au sein de toutes les cultures. L'IDDSI a ainsi abouti à un diagramme final des textures en dysphagie, composé d'un continuum de 8 niveaux (0 à 7). Ces niveaux sont identifiés par des numéros, des libellés et un code couleur [Référence : Cichero, J. A. Y., et al. (2016). Development of International Terminology and Définitions for Texture-Modified Foods and Thickened Fluids Used in Dysphagia Management: The IDDSI Framework. Dysphagia, 32(2), 293 314 (doi.org/10.1007/s00455-016-9758-y).

L'échelle IDDSI fournit ainsi une terminologie commune pour décrire les textures d'aliments et boissons plus ou moins épaissies. Les tests IDDSI permettent également de confirmer la texture d'une denrée au moment du service. Les tests doivent être effectués sur les aliments et boissons dans les conditions de service habituelles (en particulier la température). Le professionnel de santé formé a la responsabilité de faire des recommandations de texture pour un patient particulier en se basant sur son évaluation complète.

Dans le contexte de l'invention, une texture modifiée de grade 6 ou inférieur est particulièrement adaptée, plus préférentiellement une texture de grade 5.

Celle-ci correspond à une texture d'aliment « haché et lubrifié ». Selon la description de l'IDDSI, version 2022, les produits associés à cette texture doivent correspondre aux critères suivants :
- Le produit doit être tendre et lubrifié sans liquide se séparant du solide
- Il ne doit pas être nécessaire de les croquer
- Il faut pouvoir les mastiquer à minima
- La taille des particules ne dépasse pas 4 mm
- Les petits morceaux peuvent s'écraser avec la langue
- Il doit tenir en bloc sur la fourchette sans écoulement de liquide ou jus ni même de particules qui s'en échappent
- Il doit conserver sa forme lors de la prise en cuillère et tomber facilement lorsque la cuillère est inclinée ou légèrement secouée
- Il ne doit pas être trop ferme, ni trop collant

Ainsi, un produit en texture modifiée de grade 5 selon l'échelle de l'IDDSI, doit pouvoir être mastiqué pour rassembler la nourriture en une boule afin de l'emmener jusqu'à l'arrière-bouche pour l'avaler. Il est également nécessaire d'éviter que ces aliments soient trop collants et qu'ils n'adhèrent pas aux joues, dents, palais ou à la gorge. Il est donc particulièrement destiné aux personnes atteintes de troubles de la mastication et/ou de la déglutition, mais également de dysphagie mais conservant des capacités totales ou réduites à mastiquer les aliments.

Selon un autre objet particulièrement d'intérêt, le produit nutritionnel obtenu par cuisson vapeur se déforme sans reprendre sa forme initiale avec l'application d'une pression d'une valeur inférieure ou égale à 100 kPa, préférentiellement d'une valeur inférieure ou égale à 17 kPa.

Le produit nutritionnel reconstitué selon l'un des quelconques objets précédemment décrits, la poudre, soit le mélange comprenant au moins les fibres fonctionnelles, les protéines ayant des propriétés foisonnantes, le blanc d'oeuf et la farine de céréales, est préférentiellement mélangé avec un liquide, qui représente, préférentiellement entre 40 et 80% en poids du poids total du produit avant la cuisson. Plus préférentiellement, la solution liquide représente entre 50 et 80% en poids du poids total du produit avant la cuisson, ou entre 40 et 70% en poids du poids total du produit avant la cuisson. Avantageusement, la poudre selon l'invention et le procédé, permet d'obtenir, d'une part, un produit nutritionnel dit texture modifiée, soit une texture de grade 6 ou moins, selon l'échelle IDDSI, et d'autre part, d'un produit nutritionnel dit texture normale, soit très proche d'un produit dit de boulangerie classique.

Lorsque la solution liquide représente entre 50 et 80% en poids du poids total de la pâte, c'est-à-dire avant la cuisson, soit du mélange total de l'étape b) comprenant la poudre et la solution liquide avant la cuisson, le produit nutritionnel reconstitué présente une texture dites modifiée.

Lorsque la solution liquide représente entre 40 et 70% en poids du poids total de la pâte avant la cuisson, soit du mélange total de l'étape b) comprenant la poudre et la solution liquide avant la cuisson, le produit nutritionnel reconstitué présente une texture dites normale.

### Procédé

Le produit nutritionnel selon l'invention peut être obtenu par tous procédés adaptés. Préférentiellement, le produit nutritionnel selon l'invention est susceptible d'être obtenu par un procédé de préparation comprenant les étapes suivantes :
a) mélanger (i) la poudre comprenant au moins une fibre fonctionnelle, au moins une protéine foisonnante, au moins une farine de céréales ayant, préférentiellement une capacité de rétention d'eau d'au moins 100%, du blanc d'oeuf, et (ii) un liquide choisi parmi l'eau, le lait et une boisson végétale, par un organe de mélange configuré pour générer une force de cisaillement comprise entre 3000 et 12500 rpm, pendant une durée comprise entre 30 secondes et 2 minutes,
b) verser dans un moule le mélange obtenu à l'étape a), et
c) cuire au moyen d'un four vapeur ou d'un four à chaleur sèche.

Selon un autre aspect, l'invention concerne un procédé de préparation du produit nutritionnel reconstitué selon l'invention comprenant les étapes suivantes :
a) mélanger (i) la poudre comprenant au moins une fibre fonctionnelle, au moins une protéine foisonnante, au moins une farine de céréales, du blanc d'oeuf, et (ii) un liquide choisi parmi l'eau, le lait et une boisson végétale, par un organe de mélange configuré pour générer une force de cisaillement comprise entre 3000 et 12500 rpm, pendant une durée comprise entre 30 secondes et 2 minutes
b) verser dans un moule le mélange obtenu à l'étape a), et
c) cuire au moyen d'un four vapeur ou d'un four à chaleur sèche, préférentiellement au moyen d'un four vapeur.

Lorsque le procédé met en oeuvre une cuisson au moyen d'un four vapeur, notamment pour obtenir une texture dites modifiée. La cuisson au four vapeur présente préférentiellement, au moins une des caractéristiques suivantes :
- une humidité comprise entre 40 et 100%,
- une durée de cuisson comprise entre 10 et 60 minutes,
- une température comprise entre 90°C et 100°C.

Lorsque le procédé met en oeuvre une cuisson au moyen d'un four à chaleur sèche, notamment pour obtenir une texture dites normale. La cuisson en chaleur sèche présente préférentiellement, une durée de cuisson comprise entre 5 et 30 minutes à une température comprise entre 170 et 220°C.

### Utilisation

Le produit nutritionnel selon l'un des quelconques objets précédents présente une texture dite modifiée, qui est obtenue en adaptant la composition d'une part, et le procédé de cuisson d'autre part. Selon une variante, une texture dites normale peut également être obtenue par l'utilisation d'un procédé de cuisson usuel en boulangerie.

Ainsi, le produit nutritionnel selon l'invention est particulièrement adapté aux personnes présentant des risques de fausses routes, ou atteintes de troubles de la déglutition et/ou de la mastication, et/ou de dysphagie, mais également aux personnes à risque de dénutrition ou dénutries. En effet, une portion de produit nutritionnelle selon l'invention peut apporter entre 8 et 20 % des apports protéiques journaliers.

Aussi, l'invention se rapporte également à un produit nutritionnel pour son utilisation dans la prévention, prophylaxie et/ou le traitement des troubles de la mastication et/ou de la déglutition, comprenant :
- un liquide (i) choisi parmi l'eau, le lait et une boisson végétale, et
- un mélange (ii) comprenant :
   ▪ au moins une fibre fonctionnelle,
   ▪ au moins une protéine foisonnante,
   ▪ au moins une farine de céréales, préférentiellement une farine de céréales ayant une capacité de rétention d'eau d'au moins 100%, et
   ▪ du blanc d'oeuf.

De façon particulièrement préférée, l'invention se rapporte à un produit nutritionnel pour son utilisation dans la prévention et/ou le traitement de la dysphagie, encore plus préférentiellement le produit est destiné à une utilisation dans le traitement ou la prophylaxie de la dénutrition chez des patients souffrant de dysphagie, et/ou dans la nutrition de patients souffrant de dysphagie.

L'invention est à présent illustrée par des exemples non limitatifs de compositions selon l'invention et par des résultats.

### Exemples

### Exemple 1 - Pain en texture modifiée de grade 5 selon l'échelle IDDSI

Les ingrédients en poudre sont mélangés et une quantité de 50 g de poudre est reconstituée dans 110 g d'eau. L'eau et la poudre sont mélangés jusqu'à homogénéisation totale. Le mélange obtenu est introduit dans un récipient profond afin d'y introduire un mixeur plongeur à une vitesse de 12500 rpm pendant 60 secondes. Une fois la préparation mélangée, elle est coulée dans des moules silicones ronds afin d'obtenir des portions de pain. Le moule est ensuite filmé et introduit dans un four vapeur en chaleur humide avec un réglage à 100 % d'humidité. La cuisson se fait à 90°C pour une durée de 25 minutes.

Les caractéristiques du produit selon l'exemple sont détaillées dans le tableau 1 ci-après.

**[Tableau 1]**

| **Ingrédients** | **% du produit en poudre** |
|---|---|
| Farine de maïs | 19,3 |
| Fibre de pois | 4,3 |
| Poudre à lever | 2,8 |
| Sucre | 5,7 |
| Sel | 1,2 |
| Amidon de blé modifié | 2,2 |
| Peptides hydrolysés de pois | 4,6 |
| Protéines de lait | 3,2 |
| Fibre de psyllium | 3,3 |
| Fibre de lin | 0,2 |
| Levain de blé | 4,8 |
| Malt d'orge | 1,1 |
| Fibre d'acacia | 14,8 |
| Maltodextrine | 16,9 |
| Blanc d'oeuf | 15,2 |
| Arôme croûte de pain | 0,4 |

### Exemple 2 - Pain en texture modifiée de grade 5 selon l'échelle IDDSI

Les ingrédients en poudre sont mélangés et une quantité de 58 g de poudre est reconstituée dans 122 g d'eau. L'eau et la poudre sont mélangés jusqu'à homogénéisation totale. Le mélange obtenu est introduit dans un récipient profond afin d'y introduire un mixeur plongeur à une vitesse de 12500 rpm pendant 60 secondes. Une fois la préparation mélangée, elle est coulée dans des moules silicones ronds afin d'obtenir des portions de pain. Le moule est ensuite filmé et introduit dans un four vapeur en chaleur humide avec un réglage à 100 % d'humidité. La cuisson se fait à 90°C pour une durée de 35 minutes.

Les caractéristiques du produit selon l'exemple sont détaillées dans le tableau 2 ci-après.

**[Tableau 2]**

| **Ingrédients** | **% du produit en poudre** |
|---|---|
| Farine de maïs | 22,4 |
| Fibre de blé | 5,2 |
| Fibre de pois | 1,4 |
| Poudre à lever | 3,5 |
| Sucre | 6 |
| Sel | 1,5 |
| Amidon de blé modifié | 2,5 |
| Peptides hydrolysés de pois | 5,9 |
| Protéines de lait | 3,6 |
| Fibre de lin | 0,3 |
| Levain de blé | 6,2 |
| Malt d'orge | 1,5 |
| Fibre d'acacia | 12,2 |
| Maltodextrine | 13,5 |
| Blanc d'oeuf | 13,9 |
| Arôme croûte de pain | 0,4 |

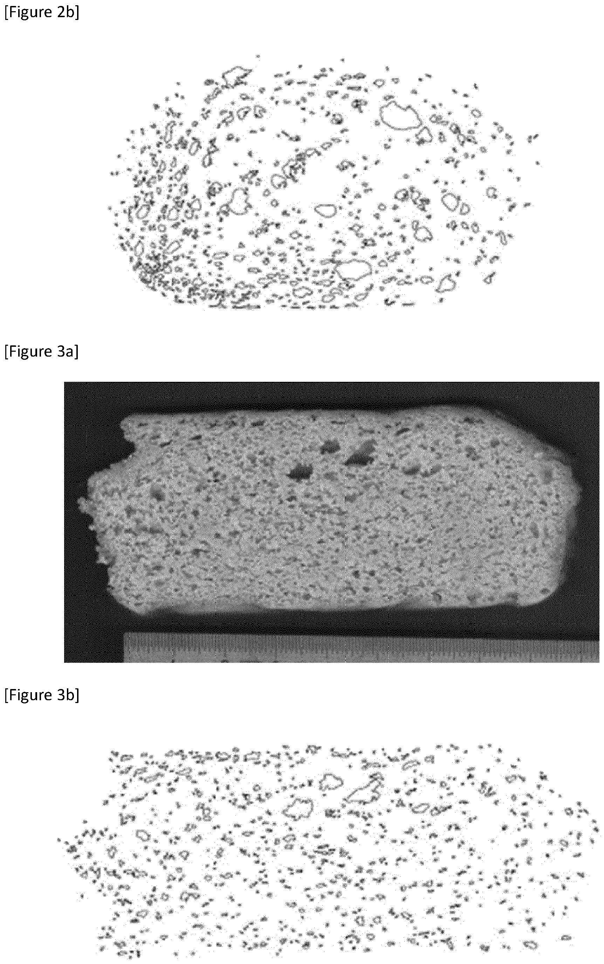

Les résultats du pain obtenu selon l'exemple 2 sont présentés en Figure 2a et Figure 2b.

### Exemple 3 - Pain en texture normale

Les ingrédients en poudre sont mélangés et une quantité de 65 g de poudre est reconstituée dans 80g d'eau. L'eau et la poudre sont mélangés jusqu'à homogénéisation totale. Le mélange obtenu est introduit dans un récipient profond afin d'y introduire un mixeur plongeur à une vitesse de 12500 rpm pendant 60 secondes. Une fois la préparation mélangée, elle est coulée dans des moules silicones ronds afin d'obtenir des portions de pain. Le moule est ensuite introduit dans un four en chaleur sèche. La cuisson se fait à 180°C pour une durée de 15 minutes.

Les caractéristiques du produit selon l'exemple sont détaillées dans le tableau 3 ci-après.

**[Tableau 3]**

| **Ingrédients** | **% du produit en poudre** |
|---|---|
| Farine de maïs | 19,3 |
| Fibre de pois | 4,3 |
| Poudre à lever | 2,8 |
| Sucre | 5,7 |
| Sel | 1,2 |
| Amidon de blé modifié | 2,2 |
| Peptides hydrolysés de pois | 4,6 |
| Protéines de lait | 3,2 |
| Fibre de psyllium | 3,3 |
| Fibre de lin | 0,2 |
| Levain de blé | 4,8 |
| Malt d'orge | 1,1 |
| Fibre d'acacia | 14,8 |
| Maltodextrine | 16,9 |
| Blanc d'oeuf | 15,2 |
| Arôme croûte de pain | 0,4 |

Les résultats du pain obtenu selon l'exemple 3 sont présentés en Figure 3a et Figure 3b.

### Exemple 4 - Pain en texture modifiée de grade 6 selon l'échelle IDDSI

Les ingrédients en poudre sont mélangés et une quantité de 65 g de poudre est reconstituée dans 130 g d'eau. L'eau et la poudre sont mélangés jusqu'à homogénéisation totale. Le mélange obtenu est introduit dans un récipient profond afin d'y introduire un mixeur plongeur à une vitesse de 12500 rpm pendant 60 secondes. Une fois la préparation mélangée, elle est coulée dans des moules silicones ronds afin d'obtenir des portions de pain. Le moule est ensuite filmé et introduit dans un four vapeur en chaleur humide avec un réglage à 100 % d'humidité. La cuisson se fait à 90°C pour une durée de 50 minutes.

Les caractéristiques du produit selon l'exemple sont détaillées dans le tableau 4 ci-après.

**[Tableau 4]**

| **Ingrédients** | **% du produit en poudre** |
|---|---|
| Farine de maïs | 16 |
| Fibre de blé | 5,5 |
| Poudre à lever | 6 |
| Sucre | 6 |
| Sel | 1 |
| Amidon de blé modifié | 2 |
| Peptides hydrolysés de pois | 6 |
| Protéines de lait | 4,5 |
| Fibre de psyllium | 1,8 |
| Fibre de lin | 0,2 |
| Levain de blé | 6 |
| Malt d'orge | 1 |
| Fibre d'acacia | 13,8 |
| Maltodextrine | 15,6 |
| Blanc d'oeuf | 14,2 |
| Arôme croûte de pain | 0,4 |

### Exemple 5 - Essai comparatif d'un pain selon l'invention et du pain G-nutrition^{®}

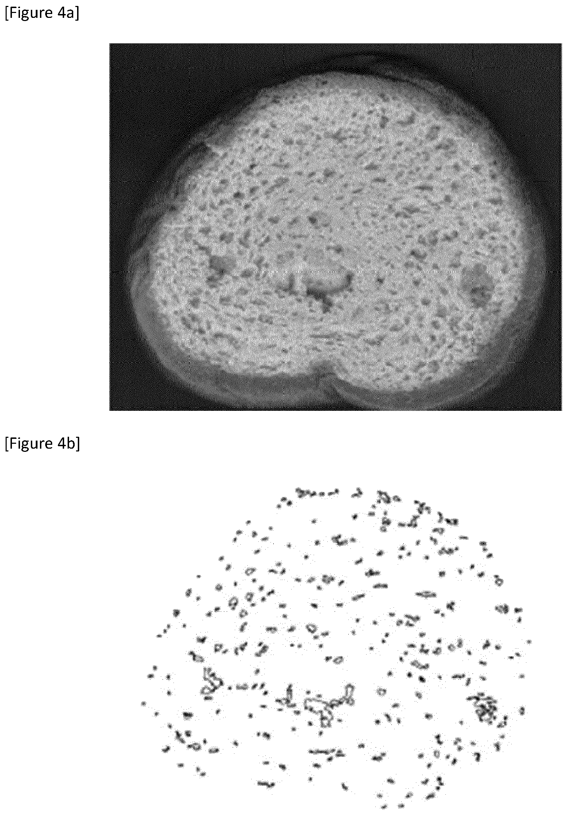

Les résultats comparatifs sont présentés dans le tableau 5 ci-après, représentant respectivement les résultats d'un traitement d'image d'une section transversale d'une portion de pain selon l'exemple 2 et d'une section transversale du pain G-nutrition^{®} (Figure 3b et Figure 4b) par le logiciel Image J.

**[Tableau 5]**

| | **Exemple 2** | **G-nutrition^{®}** | **Unité** |
|---|---|---|---|
| Surface totale de la section | 2232 | 705 | mm2 |
| Taille limite des bulles prises en compte (S°) | 0,05 | 0,05 | mm2 |
| Nombre total de bulles > S° | 554 | 308 | bulles |
| Nombre total de bulles par cm2 de pain | 24,8 | 43,7 | bulles/cm2 |
| Surface moyenne des bulles | 0,28 | 0,12 | mm2 |
| Surface totale des bulles | 228 | 70,3 | mm2 |
| % de surface de pain occupées par les bulles | 10,21 | 9,97 | % |

La portion de pain selon l'exemple 2 présente de nombreuses alvéoles pour une portion (554 bulles dénombrées) par comparaison au produit G-Nutrition^{®} (308 bulles dénombrées). Toutefois, le nombre d'alvéoles par unité de surface est plus faible 24,8 bulles/cm2 dénombrées contre 43,7 bulles/cm2 pour le produit G-Nutrition^{®} bien qu'elles occupent une surface plus importante (10,21%) par rapport au pain G-Nutrition^{®} (9,97%). En effet, les alvéoles présentes dans le pain selon l'exemple 2 sont deux fois plus grandes que le pain G-Nutrition^{®} (0,28mm2 en moyenne contre 0,12mm2). Par conséquent, le pain en texture modifiée présente de grandes alvéoles et donc une texture très aérée et mousseuse permettant d'appliquer une moindre pression avec la langue au cours de la déglutition. Aussi, le produit nutritionnel selon l'invention est particulièrement adapté aux personnes souffrant de ces troubles, en particulier de la dysphagie.

### Exemple 6 - Tests IDDSI à la fourchette et à la cuillère inclinée du produit nutritionnel selon l'exemple 2

Ces essais sont des tests effectués sur le produit selon l'exemple 2, selon les critères définis dans l'échelle IDDSI, en particulier de l'IDDSI grade 5, comprenant un test d'écrasement du produit à la fourchette avec la mesure de la taille des particules et un test à la cuillère inclinée pour évaluer la tenue du produit.

Le protocole du test est notamment décrit dans la description de l'IDDSI, version 2022 (https://www.iddsi.org/framework/food-testing-methods/).L

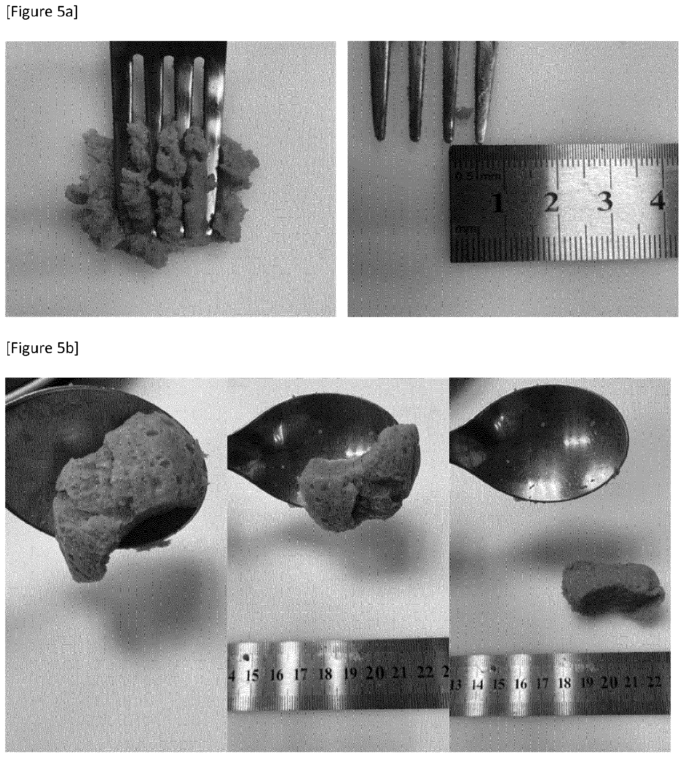

Les résultats sont présentés en Figure 5a et Figure 5b.

Comme représenté en Figure 5a, le test IDDSI à la fourchette permet d'obtenir une taille des particules ne dépassant pas 4 mm pour les adultes (2 mm pour les enfants), correspondant approximativement à l'espace entre les dents d'une fourchette.

Comme représenté en Figure 5b, le test IDDSI à la cuillère inclinée démontre que le produit selon l'exemple 2 conserve sa forme dans la cuillère et tombe facilement quand celle-ci est inclinée ou légèrement secouée. Le produit n'est ni trop ferme ni trop collant.

## Revendications

1. Produit nutritionnel pour son utilisation dans la prévention et/ou le traitement des troubles de la mastication et/ou de la déglutition, comprenant :
- un liquide (i) choisi parmi l'eau, le lait et une boisson végétale, et
- un mélange (ii) comprenant :
▪ au moins une fibre fonctionnelle choisie parmi la fibre de blé, de pois, d'avoine, d'agrume, de lin, de psyllium, de soja, de carotte, la gomme de xanthane et leurs combinaisons,
▪ au moins une protéine foisonnante choisie parmi un peptide hydrolysé de protéine végétale, une protéine d'origine animale ou végétale, et leurs combinaisons,
▪ au moins une farine de céréales,
▪ du blanc d'oeuf,
ledit produit nutritionnel présente une texture alvéolée.

2. Produit nutritionnel pour son utilisation selon la revendication précédente, pour son utilisation dans la prévention et/ou le traitement de la dysphagie.

3. Produit nutritionnel pour son utilisation selon l'une des revendications précédentes, **caractérisé en ce que** le produit nutritionnel est préparé/obtenu par un organe de mélange configuré pour générer une force de cisaillement comprise entre 3000 et 12500 rpm, adaptée pour mélanger (i) le liquide et (ii) le mélange comprenant la fibre fonctionnelle, la protéine foisonnante, la farine de céréales et le blanc d'oeuf.

4. Produit nutritionnel pour son utilisation selon la revendication précédente, **caractérisé en ce que** le produit nutritionnel est obtenu par cuisson vapeur et présente une texture modifiée de grade 5 ou 6, mesuré selon l'échelle IDDSI, préférentiellement de grade 5.

5. Produit nutritionnel pour son utilisation selon l'une des revendications précédentes, **caractérisé en ce que** le peptide hydrolysé est un peptide hydrolysé de protéine de pois et la protéine d'origine animale est une protéine de lait.

6. Produit nutritionnel pour son utilisation selon la revendication précédente, **caractérisé en ce que** le rapport peptides hydrolysés de protéine de pois et protéine de lait est compris entre 1 et 2.

7. Produit nutritionnel pour son utilisation selon la revendication précédente, **caractérisé en ce que** les peptides hydrolysés de protéine de pois représentent au plus 7% en poids du poids total de matière sèche et les protéines de lait représentent au plus 14% en poids du poids total de matière sèche.

8. Produit nutritionnel pour son utilisation selon l'une des revendications précédentes, **caractérisé en ce que** la quantité de blanc d'oeuf est comprise entre 3 et 25% en poids du poids total de matière sèche.

9. Produit nutritionnel pour son utilisation selon l'une des revendications précédentes, **caractérisé en ce que** la quantité de fibre fonctionnelle est comprise entre 2 et 12% en poids du poids total de matière sèche.

10. Produit nutritionnel pour son utilisation selon l'une des revendications précédentes, **caractérisé en ce que** la farine de céréales est une farine de maïs.

11. Produit nutritionnel pour son utilisation selon la revendication précédente, **caractérisé en ce que** la quantité de farine de maïs est comprise entre 10 et 30% en poids du poids total de matière sèche.

12. Produit nutritionnel pour son utilisation selon l'une des revendications précédentes, comprenant au moins ingrédient supplémentaire choisi parmi une gomme alimentaire, une fibre nutritionnelle, un levain, un arôme, de l'amidon, du malt d'orge, de la poudre à lever, un acide aminé, une vitamine, un minéral, un extrait végétal et leurs combinaisons.

13. Produit nutritionnel pour son utilisation selon l'une des revendications précédentes, **caractérisé en ce que** le produit nutritionnel est un produit de panification.

14. Poudre alimentaire destinée à être reconstituée dans un liquide pour obtenir un produit nutritionnel selon la revendication 1 comprenant :
- au moins une fibre fonctionnelle choisie parmi la fibre de blé, de pois, d'avoine, d'agrume, de lin, de psyllium, de soja, de carotte, la gomme de xanthane et leurs combinaisons,
- au moins une protéine foisonnante choisie parmi un peptide hydrolysé de protéine végétale, une protéine d'origine animale ou végétale, et leurs combinaisons,
- au moins une farine de céréales, et
- du blanc d'oeuf.

15. Procédé de préparation d'un produit nutritionnel comprenant les étapes suivantes :
a. mélanger la poudre selon la revendication précédente et un liquide choisi parmi l'eau, le lait et une boisson végétale, par un organe de mélange configuré pour générer une force de cisaillement comprise entre 3000 et 12500 rpm, pendant une durée comprise entre 30 secondes et 2 minutes
b. verser dans un moule le mélange obtenu à l'étape a), et
c. cuire au moyen d'un four vapeur ou d'un four à chaleur sèche ou d'un bain marie ou d'un micro-ondes.
